# EUROPÄISCHE PATENTSCHRIFT

(11) **EP 3 299 044 B1**
(45) Veröffentlichungstag und Bekanntmachung des Hinweises auf die Patenterteilung: **02.12.2020**
(21) Anmeldenummer: 16190243.2
(22) Anmeldetag: 22.09.2016
(51) Int. Cl.: A61M 1/10

(54) **MEDIZINISCHES GERÄT**
MEDICAL DEVICE
INSTRUMENT MÉDICAL

(43) Veröffentlichungstag der Anmeldung: 28.03.2018
(73) Patentinhaber: Berlin Heart GmbH, 12247 Berlin (DE)
(72) Erfinder: FRISCHKE, Michael, 15834 Rangsdorf (DE); WINTERWERBER, Kim Peter, 12357 Berlin (DE)
(74) Vertreter: Pfenning, Meinig & Partner mbB

(56) Entgegenhaltungen:
- WO-A1-2011/073334
- WO-A1-2014/161978
- US-A1- 2008 319 544

## Beschreibung

Die Neuerung liegt auf dem Gebiet der Mechanik und des Maschinenbaus und ist mit besonderem Vorteil auf dem Gebiet der Medizintechnik einsetzbar. Konkret bezieht sich die Neuerung auf ein medizinisches Gerät mit einem implantierbaren Element, das mit einer Steuereinrichtung verbunden ist, die außerhalb eines Patientenkörpers angeordnet sein kann.

Derartige Steuereinrichtungen erlauben eine mit dem Fortschreiten der Technik immer detailliertere Steuerung des implantierbaren Elements, wobei eine anspruchsvollere Steuerung üblicherweise auch mit einer weiterentwickelten Sensorik am Patientenkörper einhergeht. Auch die implantierbaren Elemente lassen teilweise eine Vielzahl von Betriebsmodi und Steuermöglichkeiten zu.

Für viele Zwecke, wie beispielsweise einen externen Zugriff auf die Steuereinrichtung, Softwareupdates und allgemein eine Kommunikation mit anderen Geräten wird an derartigen Steuereinrichtungen eine Kommunikationsschnittstelle benötigt, die oft als Funkschnittstelle mithilfe eines Funkmoduls ausgebildet ist. Beim Austatten eines medizinischen Gerätes mit einem Funkmodul sind jedoch besondere Anforderungen zu beachten, da das Senden durch den Patientenkörper und in diesen hinein aus gesundheitlichen Gründen sowie wegen möglicher Störungen implantierter Elemente vermieden werden soll. Zudem soll mit möglichst geringer Sendeleistung und minimierter Dämpfung der Funksignale eine möglichst gute und störungsfreie Kommunikation zu einem externen Transceiver aufgebaut werden.

Der vorliegenden Neuerung liegt daher vor dem Hintergrund des Standes der Technik die Aufgabe zugrunde, ein medizinisches Gerät der eingangs genannten Art zu schaffen, dessen Steuereinrichtung eine optimierte Kommunikation über eine Funkschnittstelle zulässt.

Eine weitere wichtige Randbedingung sind die strengen EMV-Anforderungen. Daher sind häufig Faraday'sche Käfige notwendig, um die Steuerelektronik zu schützen. Die Kunst besteht nun darin, eine Funkverbindung so zu implementieren, dass der EMV-Schutz nicht verlorengeht, aber dennoch gute Sende- und Empfangseigenschaften erreicht werden.

Das Dokument WO 2014/161978 A1 bezieht sich auf eine implantierbare Kabelverbindungseinrichtung mit einem Kabelverbinder, der mehrere Steckverbindungselemente aufweist, mit denen je ein implantierbares Kabel durch eine Steckverbindung verbindbar ist, wobei der Kabelverbinder als steifer Körper aus einem biokompatiblen Material gebildet ist.

Das Dokument WO 2011/073334 A1 betrifft einen implantierbaren Pulsgenerator zur Stimulation einer neurologischen Zellmasse mit einem Gehäuse, das das Pulserzeugungsmodul (PGM) zumindest teilweise umschließt und das für elektromagnetische Felder mit Radiofrequenz durchlässig ist, oder wobei das Pulserzeugungsmodul eine Controllerschaltung umfasst, die als zwei oder mehr Leiterplatten bereitgestellt wird, die zusammenwirkend verbunden sind, wobei eine solche Leiterplatte eine Schnittstellenleiterplatte ist, wobei eine Komponente der Steuerschaltung auf der Schnittstellenleiterplatte angeordnet ist, und Durchführungsdrähte für den Verbinderblock damit verbunden sind, und eine der gegenüberliegenden Oberflächen der Schnittstellenleiter platte über Öffnungen im PGM -Gehäuse für die Durchführungsdrähte ausgerichtet ist.

Das Dokument US 2008/319544 A1 offenbart ein Blutpumpensystem für ein künstliches Herz, umfassend: eine Steuereinheit zum Steuern einer Blutpumpe, wobei die Steuereinheit einen ersten Alarmerzeugungsteil zum Erzeugen eines ersten Alarmsignals gemäß dem Zustand der Blutpumpe und einen Lautsprecher zum Ausgeben eines hörbaren Signals beim Empfang des ersten Alarmsignals umfasst, wenn sich die Blutpumpe in einem anomalen Zustand befindet; einen Überwachungsabschnitt, der konfiguriert ist, den Zustand der Steuereinheit zu überwachen; einen Bestimmungsabschnitt, der konfiguriert ist zum Bestimmen auf der Grundlage des Ausgangssignals von dem Überwachungsabschnitt, ob sich die Steuereinheit in einem anomalen Zustand befindet oder nicht; und einen zweiten Alarmerzeugungsteil zum Erzeugen eines zweiten Alarmsignals gemäß dem Zustand der Steuereinheit auf der Grundlage des Ergebnisses der Bestimmung durch den Bestimmungsabschnitt.

Die Aufgabe wird mit den Merkmalen der Neuerung gemäß Patentanspruch 1 gelöst. Die Patentansprüche 2 bis 12 beschreiben besondere Implementierungen.

Demgemäß bezieht sich die Neuerung auf ein medizinisches Gerät mit einem implantierbaren Element, insbesondere einer Herzpumpe, und einer Steuereinrichtung für das implantierbare Element, die mittels einer ersten Verbindung mit dem implantierbaren Element verbunden ist. Eine besonders gute Kommunikation der Steuereinrichtung mit außerhalb des Gerätes angeordneten Elementen mittels eines Funkmoduls ergibt sich dadurch, dass die Steuereinrichtung für eine vorbestimmte Ausrichtung relativ zu einem Patientenkörper eingerichtet ist und ein Funkmodul aufweist, wobei eine Antenne des Funkmoduls derart angeordnet ist, dass der Bereich, in dem von der Steuereinrichtung aus gesehen die Position des Patientenköpers vorgesehen ist, wenigstens teilweise durch elektromagnetisch abschirmende, insbesondere elektrisch leitende Teile der Steuereinrichtung oder ihres Gehäuses von der Antenne abgeschirmt ist.

Die Antenne des Funkmoduls ist somit derart angeordnet, dass die ausgesandte elektromagnetische Strahlung, wenn die Steuereinrichtung am Patientenkörper getragen wird, diesen nicht oder nur in geringem Maß trifft. Entweder die Steuereinrichtung selbst, beispielsweise in Form von Leiterplatten oder elektronischen Bauelementen, oder Teile des Gehäuses der Steuereinrichtung sind derart gestaltet, dass sie beispielsweise elektrisch leitend sind oder elektrisch leitende Elemente enthalten und somit elektromagnetische Strahlung weitgehend abschirmen. Die Antenne des Funkmoduls ist auf der dem Patientenkörper abgewandten Seite dieser abschirmenden Teile angeordnet, so dass die elektromagnetische Strahlung den Patientenkörper nicht stärker als notwendig durchdringt.

Eine Implementierung kann dabei vorsehen, dass die Antenne derart positioniert und ausgerichtet ist, dass der Einfluss eines Patientenkörpers im Betriebszustand auf die Abstrahlcharakteristik der Antenne minimiert ist, insbesondere dadurch, dass das Maximum der Abstrahlungsintensität von der Antenne aus in einer Richtung angeordnet ist, die die Position des Patientenkörpers nicht berührt. Die Ausrichtung der Antenne kann so vorgesehen sein, dass von der Antenne aus gesehen weniger als 50 %, vorteilhaft weniger als 20 %, weiter vorteilhaft weniger als 10 % der abgestrahlten Sendeleistung den für den Patientenkörper vorgesehenen Bereich durchdringen.

Es kann beispielsweise vorgesehen sein, dass die Steuereinrichtung ein wenigstens abschnittsweise elektrisch leitendes Gehäuse aufweist. Das Gehäuse der Steuereinrichtung kann beispielsweise wenigstens teilweise aus Stahlblech oder Aluminium bestehen und entweder aus einem Stück durch Gießen oder Tiefziehen hergestellt sein oder aus mehreren plattenartigen Elementen zusammengefügt sein. Üblicherweise wird ein derartiges Gehäuse der Steuereinrichtung flüssigkeitsdicht hergestellt, so dass bei Zusammenfügen aus mehreren Teilen die Fügestellen dichtend gestaltet sind.

Hier kann außer der Flüssigkeitsdichtigkeit auch eine Dichtigkeit gegenüber elektromagnetischen Wellen im Rahmen einer elektromagnetischen Verträglichkeit angestrebt werden. Beispielsweise können dort eingesetzte Fügestoffe oder Elastomerdichtungen mit leitenden Partikeln durchsetzt sein, um eine elektromagnetische Dichtigkeit zu gewährleisten.

Die elektromagnetisch abschirmenden Teile des Gehäuses können beispielsweise auch als Kunststoffteile ausgebildet sein, wobei der Kunststoff ausreichend mit leitenden Partikeln gefüllt sein muss.

Es kann in einer Ausführungsform auch vorgesehen sein, dass die Antenne wenigstens teilweise innerhalb des Gehäuses der Steuereinrichtung hinter einem für Funksignale durchlässigen Bereich des Gehäuses oder hinter einer fensterartigen Ausnehmung des Gehäuses angeordnet ist. In diesem Fall besteht das Gehäuse der Steuereinrichtung nicht vollständig aus einem elektrisch leitenden Stoff, sondern es sind Bereiche vorgesehen, in denen das Gehäuse aus einem für Funksignale / elektromagnetische Wellen durchlässigen Werkstoff besteht oder eine Fensteröffnung aufweist. Ein solcher Teil des Gehäuses kann beispielsweise als Gehäusedeckel oder als Verschluss für eine Gehäuseöffnung vorgesehen sein. Es entsteht somit ein Fenster für elektromagnetische Wellen, hinter dem die Antenne zur Abstrahlung von Funksignalen angeordnet werden kann.

Es kann in einer Ausführungsform auch vorgesehen sein, dass die Antenne wenigstens teilweise außerhalb des Gehäuses der Steuereinrichtung angeordnet ist. In diesem Fall braucht das Gehäuse keine für elektromagnetische Wellen durchlässigen Bereiche aufzuweisen, sondern lediglich eine Durchführung für die Antennenleitung. Die Antenne selbst kann dann außen isoliert vom Gehäuse angeordnet werden.

Beispielsweise kann vorgesehen sein, dass die Antenne wenigstens abschnittsweise im Wesentlichen parallel zu einer Gehäusekante eines elektrisch leitenden Gehäuseteils der Steuereinrichtung beispielsweise in einem Abstand von wenigstens 1 mm von dieser verläuft. Wenigstens ein Abschnitt der Antenne kann somit parallel zu einer Gehäusekante und im Abstand von wenigen Millimetern von dieser vorgesehen sein, jedoch in einem Mindestabstand vom Gehäuse, der die Kapazität zum Gehäuse begrenzt und die Isolierfestigkeit zum Gehäuse sicherstellt.

Es können auch verschiedene Abschnitte der Antenne in einem Winkel von 90° entlang verschiedener Gehäusekanten im Abstand von diesen verlaufen. Damit wird sichergestellt, dass die Abstrahlcharakteristik der Antenne eine Abstrahlung der Funksignale in alle Richtungen mit einer ausreichenden Intensität ermöglicht.

Es kann in einer anderen Ausführungsform auch vorgesehen sein, dass die Antenne die Form eines Dipols, insbesondere mit zwei geschlossenen Leiterschleifen oder Leiterflächen, aufweist. Derartige Dipolschleifen können beispielsweise unterhalb eines elektrisch isolierenden, für elektromagnetische Wellen durchlässigen Bereichs des Gehäuses vorgesehen oder in derartige Gehäuseteile integriert sein. Solche Dipole können beispielsweise in Kunststoffteile des Gehäuses eingegossen sein.

Eine weitere Ausführungsform kann zu diesem Zweck beispielsweise vorsehen, dass das Gehäuse der Steuereinrichtung wenigstens einen Bereich aufweist, der aus einem elektrisch isolierenden, für elektromagnetische Wellen durchlässigen Material besteht.

Es kann, wie oben bereits teilweise erläutert, auch vorgesehen sein, dass innerhalb des Gehäuses in oder unmittelbar hinter dem aus einem elektrisch isolierenden, für elektromagnetische Wellen durchlässigen Material bestehenden Bereich eine Antenne angeordnet ist.

Eine weitere Ausführungsform kann beispielsweise vorsehen, dass der oder ein aus einem elektrisch isolierenden, für elektromagnetische Wellen durchlässigen Material bestehende(r) Bereich den elektrisch leitenden Bereich des Gehäuses vollständig oder teilweise umgibt und/oder mit dem elektrisch leitenden Bereich des Gehäuses dicht zusammengefügt, insbesondere verklebt, vergossen oder mittels einer Elastomerdichtung dicht verbunden ist. Die Antenne kann in diesem Fall beispielsweise außerhalb der Kontur der elektrisch leitenden, für elektromagnetische Wellen undurchlässigen Teile des Gehäuses, jedoch innerhalb der Kontur der für elektromagnetische Wellen durchlässigen Teile des Gehäuses liegen und somit gegen Einflüsse von außen geschützt sein.

Das Gehäuse kann beispielsweise auch eine geometrisch einfache Form, wie eine Quader- oder Würfelform, aufweisen, bei der lediglich ein Teil aus Metall und ein Teil aus Kunststoff besteht. Die Trennlinie zwischen dem Teil des Gehäuses, das aus für elektromagnetische Wellen undurchlässigem Material besteht, und dem Teil, der aus für elektromagnetische Wellen durchlässigem Material besteht, sollte dann so gewählt werden, dass der Patientenkörper gegenüber den Funksignalen, jedoch insbesondere auch gegen elektromagnetische Wellen, die von anderen elektrischen Teilen der Steuereinrichtung ausgesandt werden, geschützt ist. Befindet sich beispielsweise eine Motorsteuerung in der Steuereinrichtung, so kann diese eine Wechselrichteransteuerung mit Pulsweitenmodulation aufweisen, die hochfrequente Schaltvorgänge vornimmt, wobei durch diese Steuerung ein relativ hoher Anteil an elektromagnetischer Strahlung ausgesandt wird. Auch diese Strahlung soll durch das Gehäuse der Steuerung vom Patientenkörper abgeschirmt werden.

Es kann in einer weiteren Ausführung auch beispielsweise vorgesehen sein, dass der oder ein aus einem elektrisch Isolierenden, für elektromagnetische Wellen durchlässigen Material bestehende(r) Bereich des Gehäuses über die Kontur der elektrisch leitenden Bereiche des Gehäuses hinausragt, wobei in dem über die Kontur der elektrisch leitenden Bereiche des Gehäuses hinausragenden Bereich eine Antenne angeordnet ist.

Ebenfalls kann vorgesehen sein, dass das Funkmodul in eine Steckvorrichtung am Gehäuse der Steuereinrichtung einsteckbar ist, wobei insbesondere das Gehäuse der Steuereinrichtung eine Ausnehmung zur wenigstens teilweisen, insbesondere vollständigen Aufnahme des Funkmoduls aufweist. In diesem Fall kann beispielsweise wenigstens ein Teil des Volumens des Funkmoduls oder das gesamte Funkmodul in der Kontur des Gehäuses aufgenommen werden. Innerhalb der Ausnehmung für das Funkmodul kann beispielsweise eine Steckverbindung zur elektrischen Verbindung des Funkmoduls mit den übrigen Teilen der Steuereinrichtung vorgesehen sein. Die Ausnehmung im Gehäuse kann beispielsweise vollständig von einer metallischen Gehäusewand gebildet sein, da das Funkmodul selbst die Antenne enthalten kann und außerhalb der metallischen Teile des Gehäuses an dieses angesteckt wird. Es kann jedoch auch die Antenne dauerhaft mit dem Gehäuse der Steuereinrichtung verbunden sein und erst durch Anstecken des Funkmoduls elektrisch mit diesem verbunden werden. Mittels der elektrischen Steckverbindung des Funkmoduls wird dieses einerseits mit Energie aus dem Inneren des Gehäuses über ein Netzteil oder eine Batterie versorgt, und andererseits empfängt es Signale von den übrigen Teilen der Steuereinrichtung, die es über seine Funkschnittstelle weitergeben kann. Andererseits kann das Funkmodul auch Signale von außen empfangen und diese an die übrigen Teile der Steuereinrichtung weitergeben.

Im Folgenden wird die Neuerung anhand von Ausführungsbeispielen in Figuren einer Zeichnung gezeigt und nachfolgend erläutert. Dabei zeigt
- Fig. 1: in einer Seitenansicht eine Steuereinrichtung eines medizinischen Gerätes an einem Patientenkörper,
- Fig. 2: ein Steuergerät sowie ein implantierbares Element eines medizinischen Gerätes,
- Fig. 3: schematisch eine Steuereinrichtung mit einem Gehäuse und einer außenliegenden Antenne,
- Fign. 4, 5, 6: jeweils ein Gehäuse mit einem für elektromagnetische Wellen durchlässigen Bereich und mit einer Antenne,
- Fign. 7, 8, 9: jeweils eine Steuereinrichtung mit einer außerhalb des Gehäuses angeordneten Antenne,
- Fig. 10: eine Steuereinrichtung mit einem Gehäuse, das zusätzlich teilweise durch ein Zusatzgehäuse aus einem für elektromagnetische Wellen durchlässigen Material abgedeckt ist, und eine Antennenanordnung,
- Fign. 11, 12: jeweils ein Gehäuse mit einer für elektromagnetische Wellen durchlässigen Fensteröffnung und eine innerhalb des Gehäuses angeordnete Antenne,
- Fig. 13: eine Steuereinrichtung mit einem einsteckbaren Funkmodul,
- Fig. 14: eine Steuereinrichtung mit einem ersten Gehäuse, das von einem zweiten, für elektromagnetische Wellen durchlässigen Gehäuse vollständig umgeben ist, sowie
- Fig. 15: ein Gehäuse, das mit einem für elektromagnetische Wellen durchlässigen Stoff beschichtet ist, wobei eine Antenne in der Beschichtung angeordnet ist.

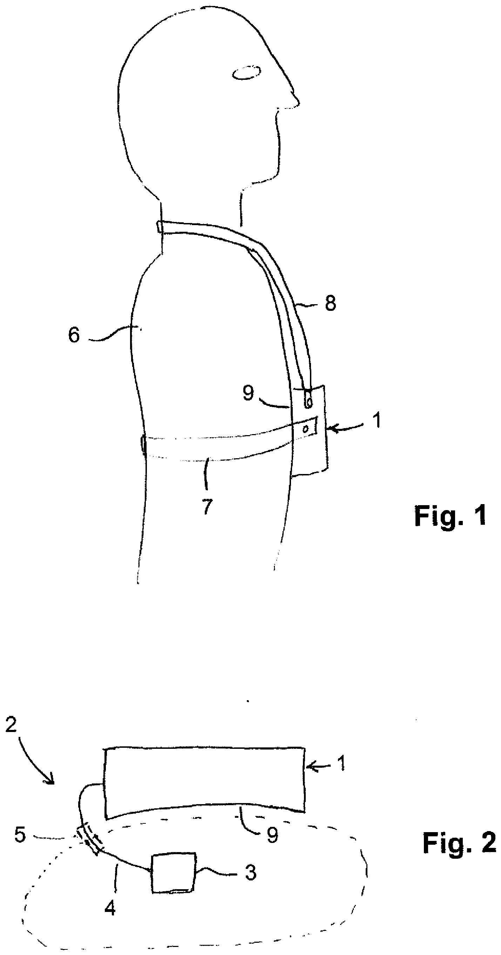

Figur 1 zeigt in einer Seitenansicht schematisch einen Patientenkörper 6 mit einer Steuereinrichtung 1, die der Patient am Körper trägt und die durch zwei beispielhaft angedeutete Tragegurte 7, 8 gehalten ist. Die Steuereinrichtung ist derart eingerichtet und mit den Gurten 7, 8 verbunden, dass die Seite 9 der Steuereinrichtung 1, die am Patientenkörper 6 anliegt, festgelegt ist. Die Seite 9 der Steuereinrichtung 1 kann auch durch andere Maßnahmen, wie beispielsweise eine besondere Beschriftung oder Beschichtung, dazu eingerichtet sein, am Patientenkörper anzuliegen. Zudem kann die Formgebung der Seite 9 der Steuereinrichtung komplementär an die Körperform des Patienten angepasst sein, beispielsweise dadurch, dass die Seite 9 in wenigstens einer, insbesondere auch zwei Ebenen, konkav ausgebildet ist.

Die Steuereinrichtung 1 ist beispielsweise mittels eines Kabels 4 mit einem implantierbaren Element 3 verbunden, das beispielsweise als Herzpumpe ausgebildet sein kann. Das Kabel 4 ist mittels einer Durchführung 5 durch die Haut des Patienten durchgeführt. Die Durchführung 5 kann beispielsweise auch unmittelbar in ein Blutgefäß des Patienten hineinführen.

Das Kabel 4 dient zum Austausch von elektrischen Signalen zwischen der Steuereinrichtung 1 und dem implantierbaren Element 3, jedoch auch zur Energieversorgung des implantierbaren Elements 3.

Figur 2 zeigt ein medizinisches Gerät 2 mit einer Steuereinrichtung 1 und einem implantierbaren Element 3 in Form einer symbolisch dargestellten Herzpumpe sowie einer Verbindung 4 in Form eines Kabels mit einer Durchführung 5 zum Durchführen durch die Haut eines Patienten.

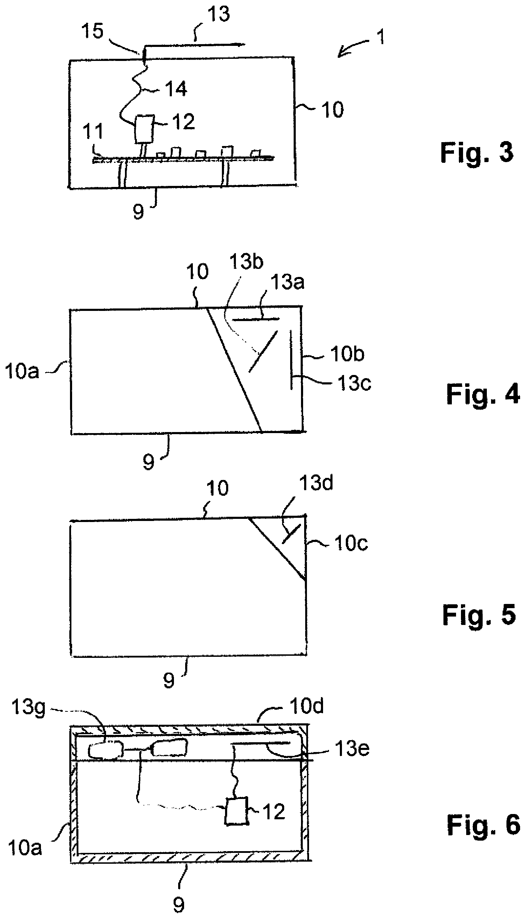

In Figur 3 ist schematisch ein Gehäuse 10 der Steuereinrichtung 1 gezeigt, das aus einem für elektromagnetische Strahlung nicht durchlässigen Material, typischerweise einem Metall, wie beispielsweise Stahl- oder Eisenblech oder Aluminium, besteht. Das Gehäuse 10 kann aber beispielsweise auch aus einem mit Metallpartikeln gefüllten Kunststoffmaterial bestehen.

Innerhalb des Gehäuses 10 ist eine Leiterplatte 11 schematisch gezeigt, auf der Bauelemente angedeutet sind.

Die elektronischen Komponenten der Steuereinrichtung sind mit einem Funkmodul 12 verbunden, das zur Übermittlung und/oder zum Empfang von Signalen mittels Funk über eine Antenne 13 dient. Unter Funk werden in diesem Zusammenhang alle Signalübermittlungen durch elektromagnetische Wellen verstanden, beispielsweise unter Verwendung bekannter Kommunikationsstandards wie Bluetooth, ZigBee, WLAN, GPRS, GSM, LTE usw.

Die Antenne 13 ist außerhalb des Gehäuses 10 an dessen der Seite 9 abgewandten Seite angeordnet, so dass der Patientenkörper, der an der Seite 9 des Gehäuses 10 anliegt, durch das Gehäuse 10 von der Antenne 13 abgeschirmt ist. Die Zuleitung 14 zur Antenne 13 ist in einer Durchführung 15 elektrisch isoliert durch das Gehäuse 10 durchgeführt. Die Antenne 13 wird üblicherweise in nur geringem Abstand von der Gehäusewand des Gehäuses 10 verlaufen, um platzsparend angeordnet zu werden und gleichzeitig nicht mit dem Gehäuse 10 leitend verbunden zu sein.

Figur 4 zeigt, dass Antennen, die dort schematisch dargestellt sind, auch innerhalb des Gehäuses 10 angeordnet sein können. Das Gehäuse 10 ist zu diesem Zweck geteilt in einen Bereich 10a des Gehäuses, der Wände aufweist, die aus einem für elektromagnetische Strahlung undurchlässigen Material, beispielsweise einem Metall, bestehen, und einen Teil 10b, dessen Wände aus einem für elektromagnetische Wellen durchlässigen Material, beispielsweise einem Kunststoff, bestehen.

Der Gehäuseteil 10b kann nach Art eines Gehäusedeckels oder einer Kappe auf den Gehäuseteil 10a aufgesetzt sein. Der Gehäuseteil 10b kann mit dem Gehäuseteil 10a beispielsweise verschweißt, verklebt oder vergossen sein. Der Gehäuseteil 10b kann auch mittels einer eingefügten Elastomerdichtung gegenüber dem Gehäuseteil 10a abgedichtet sein.

In Figur 4 sind drei mögliche Positionen 13a, 13b, 13c der Antenne angedeutet. Weitere Positionen und Ausrichtungen sind denkbar.

Figur 5 zeigt wie Figur 4 eine Aufteilung des Gehäuses 10 in zwei Teile, wobei eine Kappe 10c lediglich eine Gehäuseecke des Gesamtgehäuses 10 darstellt. Im Bereich der Gehäusekappe 10c ist eine Antenne 13d in einer möglichen Position eingezeichnet. Die Antennen 13a, 13b, 13c, 13d sind in den Figuren 4 und 5 derart dargestellt, dass in diesem Bereich die Gehäusewand der Gehäuseteile 10b, 10c für den Betrachter als durchsichtig anzunehmen ist.

Figur 6 zeigt im Querschnitt ein geteiltes Gehäuse mit einer Kappe 10d aus einem für elektromagnetische Wellen durchlässigen Material. Im Bereich der Kappe 10d sind zwei mögliche Antennenkonstellationen dargestellt, die beide jeweils mit einem Funkmodul 12 verbunden sind, und zwar einerseits die Stabantenne 13e, andererseits eine Dipolantenne 13g mit zwei Schleifen. Die Antennen 13e, 13g stellen alternative Möglichkeiten dar, wobei im Anwendungsfall nur eine der beiden Antennen vorgesehen sein wird.

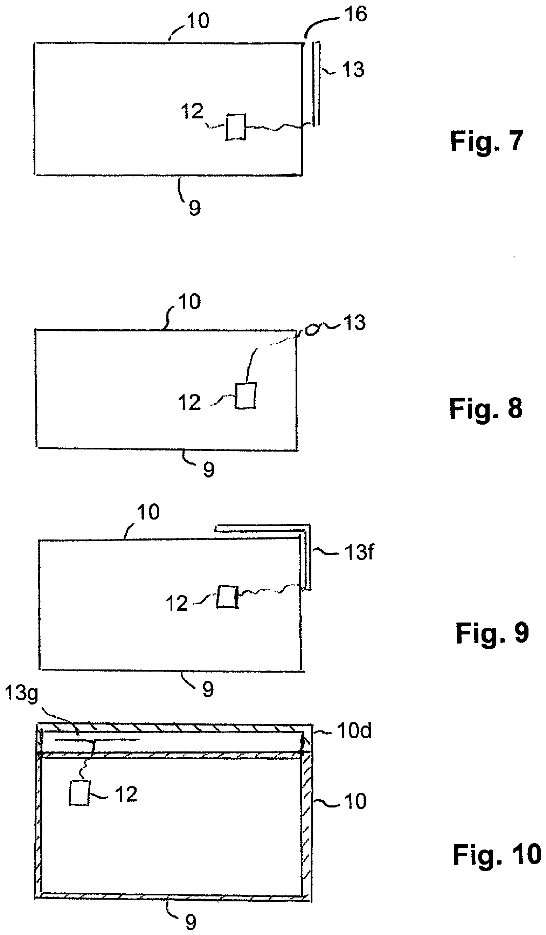

In den Figuren 7, 8 ist ein Funkmodul 12 jeweils innerhalb eines Gehäuses 10 dargestellt, das vollständig aus einem für elektromagnetische Wellen undurchlässigen Material, beispielsweise Metall, besteht, wobei das Funkmodul 12 jeweils mit einer außen am Gehäuse 10 befestigten Stabantenne 13 verbunden ist. Die Stabantenne 13 ist in den Figuren 7, 8 aus verschiedenen Blickwinkeln dargestellt. Die Stabantenne 13 befindet sich außen am Gehäuse 10 und verläuft in einem geringen Abstand, beispielsweise in einem Abstand zwischen 0,5 mm und 3 mm, von einer Gehäusekante 16 des Gehäuses 10 parallel zu dieser.

In Figur 9 ist eine Antenne 13f gezeigt, die aus zwei Abschnitten besteht, wobei jeder der Abschnitte parallel zu einer Gehäusekante des Gehäuses 10 verläuft und wobei die Abschnitte der Antenne 13f in einem rechten Winkel zueinander verlaufen. Hierdurch wird eine optimierte, möglichst zu allen Seiten gleichmäßige verteilte Strahlcharakteristik der Antenne erreicht.

Figur 10 zeigt eine Konstellation, bei der ein Gehäuse 10 der Steuereinrichtung vollständig aus einem für elektromagnetische Wellen undurchlässigen Material besteht. Ein Funkmodul 12 ist innerhalb des Gehäuses 10 angeordnet und mit einer Dipolantenne 13g verbunden, die außerhalb des Gehäuses 10 angeordnet ist. Auf das Gehäuse 10 ist eine gesonderte Kappe 10d aus einem für elektromagnetische Wellen durchlässigen Material aufgesetzt. Die Antenne 13g ist in dem Zwischenraum zwischen der für elektromagnetische Wellen / Funksignale undurchlässigen Wand des Gehäuses 10 und der Gehäusewand der Kappe 10d angeordnet. Hierdurch ist die Antenne einerseits nicht in der Abstrahlung von Signalen behindert und andererseits vor Umwelteinflüssen optimal geschützt.

Die Kappe 10d kann auf das Gehäuse 10 aufgeklebt, aufgegossen oder mittels einer Elastomerdichtung mit dem Gehäuse 10 verbunden sein. Die Kappe 10d kann auf das Gehäuse 10 mechanisch kraftschlüssig aufsteckbar oder mittels einer Schraubverbindung mit dem Gehäuse 10 verbindbar sein.

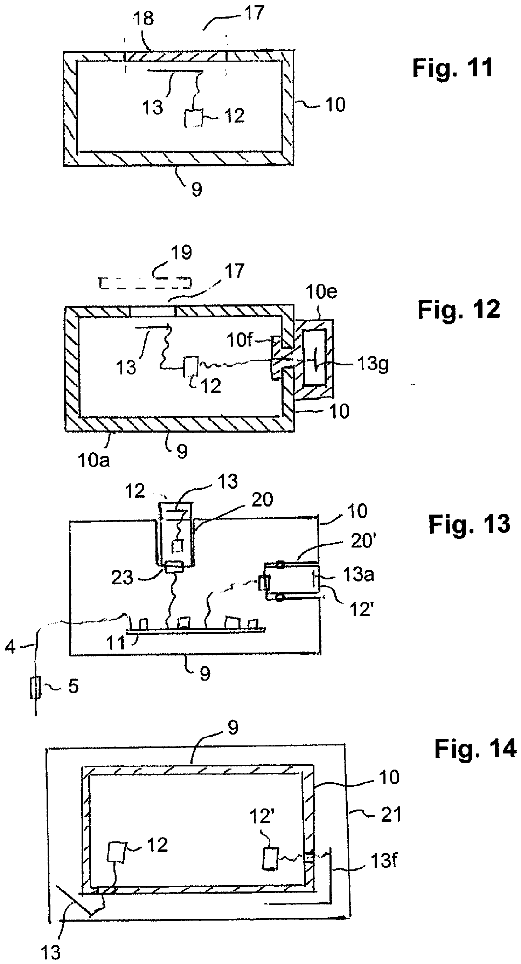

Figur 11 zeigt ein Gehäuse 10, das überwiegend aus einem Metall besteht. Innerhalb des Gehäuses 10 sind ein Funkmodul 12 sowie eine Antenne 13 vorgesehen. Auf der der Seite 9 des Gehäuses 10 abgewandten Gehäuseseite ist ein Fenster 17 vorgesehen, in dem die Gehäusewand des Gehäuses 10 durch ein für elektromagnetische Wellen durchlässiges Material 18 ausgefüllt ist. Das Fenster 17 kann beispielsweise ein rechteckiger oder runder Ausschnitt in dem beispielsweise metallischen Material des Gehäuses 10 sein, wobei der Ausschnitt mit dem Material 18, beispielsweise Kunststoff, vergossen oder in anderer Weise gefüllt sein kann. Beispielsweise kann ein komplementär geformtes Bauteil aus Kunststoff in das Fenster 17 eingeklebt, eingeklemmt und gegebenenfalls mittels einer Elastomerdichtung dicht eingefügt ein. Im Bereich hinter dem Fenster 17 und dem Materialteil 18 aus einem für elektromagnetische Wellen durchlässigen Material ist die Antenne 13 derart angeordnet, dass sie durch das Fenster 17 nach außen Funksignale abstrahlen kann.

Figur 12 zeigt ein Gehäuse 10 mit einem Gehäuseteil 10a aus Metall, der ein Fenster 17 aufweist. Im Inneren des Gehäuses 10 ist hinter dem Fenster 17 eine Antenne 13 angeordnet, die mit dem Funkmodul 12 verbunden ist. Das Fenster 17 kann beispielsweise in dem Gehäuse 10 offen bleiben; es kann jedoch auch mit einem Deckel 19 aus einem für elektromagnetische Wellen durchlässigen Material verschlossen sein, wobei der Deckel außen auf das Gehäuse 10a derart aufgelegt wird, dass er das Fenster 17 verschließt. Der Deckel 19 kann dabei auf das Gehäuse aufgeklebt oder mittels einer Schraubverbindung mit diesem verschraubt sein, wobei eine Dichtung mittels einer eingefügten Elastomerdichtung vorgesehen sein kann.

In einer weiteren Alternative ist in Figur 12 eine weitere Anordnung der Antenne 13g als Dipolantenne dargestellt, die in einem auf das Gehäuseteil 10a aufgesetzten weiteren Gehäuseteil 10e angeordnet ist, wobei der Gehäuseteil 10e aus einem Kunststoff oder einem Elastomer bestehen kann. Der Gehäuseteil 10e erstreckt sich durch eine Öffnung im Gehäuse 10a in dessen Inneres hinein und ist dort mit einem Halteflansch 10f verbunden. Dieser kann aus dem gleichen Elastomer wie der Rest des Gehäuseteils 10e bestehen und zum Einbringen in das Gehäuse 10 komprimiert oder verformt werden, so dass an dem Gehäuseteil 10e ein Hinterschnitt entsteht, mit dem das Gehäuse 10e im Gehäuseteil 10a befestigt ist.

Der Gehäuseteil 10e weist eine Durchführung für die Zuleitung von dem Funkmodul zur Antenne 13g auf. Die Antenne 13g ist somit außerhalb des elektrisch leitenden / für elektromagnetische Wellen undurchlässigen Gehäuseteils 10a angeordnet, jedoch gegen äußere Einflüsse durch den Gehäuseteil 10e geschützt.

Figur 13 zeigt in zwei Alternativen ein Gehäuse 10, das aus einem für elektromagnetische Wellen undurchlässigen Material bestehen kann und das wenigstens eine Ausnehmung 20, 20' zur Aufnahme eines einsteckbaren Funkmoduls 12, 12' aufweist. Das Funkmodul ist derart in die Ausnehmung 20, 20' einsteckbar, dass das überwiegende Volumen des Funkmoduls 12 in der Ausnehmung aufgenommen wird, so dass weniger als die Hälfte des Funkmoduls über die Kontur des Gehäuses 10 hinaussteht. Im Fall des Moduls 12' ist dieses komplementär zu der Ausnehmung 20' so ausgebildet, dass es im eingesteckten Zustand mit der Außenkontur des Gehäuses 10 abschließt.

Das Funkmodul 12, 12' weist ein Gehäuse auf, das aus einem für elektromagnetische Wellen durchlässigen Material besteht. Die Antenne 13, 13a wird innerhalb des Funkmoduls 12, 12' entweder außerhalb des Gehäuses 10 oder im Bereich der Gehäusewand der Gehäuses 10 angeordnet. Die Antenne 13, 13a ist durch das Gehäuse 10 oder beispielsweise auch durch die Platine 11 von dem auf der Seite 9 des Gehäuses 10 angeordneten Patientenkörper abgeschirmt.

In Figur 13 ist beispielhaft auch die Leitung 4 mit der Durchführung 5 dargestellt, die im Inneren des Gehäuses 10 mit der Elektronik der Steuereinrichtung, insbesondere mit der Platine 11 verbunden ist.

Figur 14 stellt eine besondere Ausführungsform der Steuereinrichtung mit einem innenliegenden Gehäuse 10 aus Metall dar, in dem beispielsweise ein Funkmodul 12, 12' angeordnet ist. Die Zuleitung zur jeweiligen Antenne 13, 13f ist durch die Gehäusewand des Gehäuses 10 isoliert durchgeführt. Die jeweils mit dem Funkmodul 12, 12' verbundene Antenne 13, 13f liegt außerhalb des Gehäuses 10, jedoch innerhalb eines Gehäuses 21, das das Gehäuse 10 vollständig umgibt, wobei das Gehäuse 21 aus einem für elektromagnetische Wellen durchlässigen Material, insbesondere einem Kunststoff, besteht. Es sind verschiedene Positionen der Antennen 13, 13f angedeutet. Mit 9 ist die dem Patientenkörper zugewandte Seite des Gehäuses 21 bzw. des Gehäuses 10 bezeichnet.

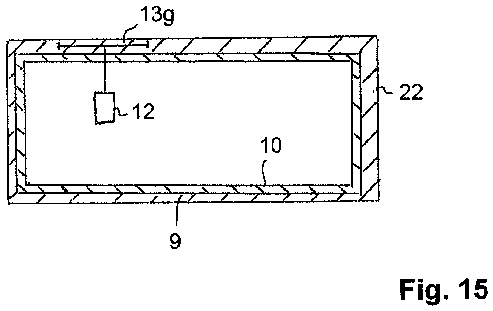

Figur 15 zeigt eine Ausführungsform, bei der ein Gehäuse 10 aus einem für elektromagnetischen Wellen undurchlässigen Material mit einem für elektromagnetische Wellen durchlässigen Material beschichtet ist. Innerhalb des Gehäuses 10 ist ein Funkmodul 12 angeordnet, das mit einer Dipolantenne 13g verbunden ist. Anstelle der Dipolantenne kann auch eine Stabantenne in verschiedenen Formen vorgesehen sein. Die Antennen sind jeweils in das Material der Beschichtung 22 eingebettet, beispielsweise eingegossen oder eingeschäumt. Das Material der Beschichtung 22 kann beispielsweise als Kunststoff, insbesondere als Schaumstoff, und/oder auch als Elastomer ausgebildet sein. Innerhalb der Beschichtung 22 ist die jeweilige Antenne 13g vom Material des Gehäuses 10, beispielsweise einem Metall, elektrisch isoliert.

Mit den oben erläuterten Ausführungsformen kann eine Antenne eines Funkmoduls bei einem medizinischen Gerät vorteilhaft in oder an einem Gehäuse einer Steuereinrichtung angeordnet werden, wobei einerseits eine Abschirmung des Patientenkörpers gegenüber der Antenne gegeben ist und andererseits die Antenne vor äußeren Einflüssen gut geschützt ist. Gleiches gilt beispielsweise für die Anordnung der Antenne eines GPS-Moduls, das in ein Steuergerät integriert sein kann.

## Patentansprüche

1. Medizinisches Gerät (2) mit einem implantierbaren Element (3), insbesondere einer Herzpumpe, und einer Steuereinrichtung (1) für das implantierbare Element, die mittels einer ersten Verbindung (4) mit dem implantierbaren Element verbunden ist, **dadurch gekennzeichnet, dass** die Steuereinrichtung (1) für eine vorbestimmte Ausrichtung außerhalb und relativ zu einem Patientenkörper (6) eingerichtet ist und ein Funkmodul (12, 12') aufweist, wobei eine Antenne (13, 13a, 13b, 13c, 13d, 13e, 13f, 13g) des Funkmoduls (12, 12') derart angeordnet ist, dass der Bereich, in dem von der Steuereinrichtung (1) aus gesehen die Position des Patientenköpers vorgesehen ist, wenigstens teilweise durch elektromagnetisch abschirmende, insbesondere elektrisch leitende Teile (10, 10a, 11) der Steuereinrichtung oder ihres Gehäuses (10, 10a, 10b, 10c, 10d, 18, 19, 21, 22) von der Antenne (13, 13a, 13b, 13c, 13d, 13e, 13f, 13g) abgeschirmt ist.

2. Medizinisches Gerät nach Anspruch 1, **dadurch gekennzeichnet, dass** die Antenne derart positioniert und ausgerichtet ist, dass der Einfluss eines Patientenkörpers im Betriebszustand auf die Abstrahlcharakteristik der Antenne minimiert ist, insbesondere dadurch, dass das Maximum der Abstrahlungsintensität von der Antenne aus in einer Richtung angeordnet ist, die die Position des Patientenkörpers nicht berührt.

3. Medizinisches Gerät nach Anspruch 1 oder 2, **dadurch gekennzeichnet, dass** die Steuereinrichtung (1) ein wenigstens abschnittsweise elektrisch leitendes Gehäuse (10, 10a, 10b, 10c, 10d) aufweist.

4. Medizinisches Gerät nach Anspruch 2 oder 3, **dadurch gekennzeichnet, dass** die Antenne wenigstens teilweise innerhalb des Gehäuses (10, 10a, 10b, 10c, 10d, 18, 19, 21, 22) der Steuereinrichtung hinter einem für Funksignale durchlässigen Bereich (10b, 10c, 10d, 18, 19) des Gehäuses oder hinter einer fensterartigen Ausnehmung (17) angeordnet ist.

5. Medizinisches Gerät nach Anspruch 1 oder einem der folgenden, **dadurch gekennzeichnet, dass** die Antenne (13, 13a, 13b, 13c, 13d, 13e, 13f, 13g) wenigstens teilweise außerhalb des Gehäuses (10, 10a, 10b, 10c, 10d, 18, 19) der Steuereinrichtung angeordnet ist.

6. Medizinisches Gerät nach Anspruch 5, **dadurch gekennzeichnet, dass** die Antenne (13, 13a, 13b, 13c, 13d, 13e, 13f, 13g) wenigstens abschnittsweise im Wesentlichen parallel zu einer Gehäusekante eines elektrisch leitenden Gehäuseteils (10a) der Steuereinrichtung in einem Abstand von wenigstens 1 mm von dieser verläuft.

7. Medizinisches Gerät nach Anspruch 5, **dadurch gekennzeichnet, dass** die Antenne (13g) die Form eines Dipols, insbesondere mit zwei geschlossenen Leiterschleifen oder Leiterflächen, aufweist.

8. Medizinisches Gerät nach Anspruch 1 oder einem der folgenden, **dadurch gekennzeichnet, dass** das Gehäuse (10, 10a, 10b, 10c, 10d, 18, 19, 21, 22) der Steuereinrichtung wenigstens einen Bereich (10b, 10c, 10d, 18, 19) aufweist, der aus einem elektrisch isolierenden, für elektromagnetische Wellen durchlässigen Material besteht.

9. Medizinisches Gerät nach Anspruch 8, **dadurch gekennzeichnet, dass** innerhalb des Gehäuses (10, 10a, 10b, 10c, 10d, 18, 19) in oder unmittelbar hinter dem aus einem elektrisch isolierenden, für elektromagnetische Wellen durchlässigen Material bestehenden Bereich (10b, 10c, 10d, 18, 19, 21, 22) eine Antenne (13, 13a, 13b, 13c, 13d, 13e, 13f, 13g) angeordnet ist.

10. Medizinisches Gerät nach Anspruch 8 oder 9, **dadurch gekennzeichnet, dass** der oder ein aus einem elektrisch isolierenden, für elektromagnetische Wellen durchlässigen Material bestehende(r) Bereich (21, 22) den elektrisch leitenden Bereich des Gehäuses vollständig umgibt und/oder mit dem elektrisch leitenden Bereich (10a) des Gehäuses dicht zusammengefügt, insbesondere verklebt, vergossen oder mittels einer Elastomerdichtung dicht verbunden ist.

11. Medizinisches Gerät nach Anspruch 8, 9 oder 10, **dadurch gekennzeichnet, dass** der oder ein aus einem elektrisch isolierenden, für elektromagnetische Wellen durchlässigen Material bestehende(r) Bereich (10b, 10c, 10d, 18, 19, 21, 22) des Gehäuses über die Kontur der elektrisch leitenden Bereiche (10a) des Gehäuses hinausragt, wobei in dem über die Kontur der elektrisch leitenden Bereiche des Gehäuses hinausragenden Bereich eine Antenne (13, 13a, 13b, 13c, 13d, 13e, 13f, 13g) angeordnet ist.

12. Medizinisches Gerät nach Anspruch 1 oder einem der folgenden, **dadurch gekennzeichnet, dass** das Funkmodul (12, 12') in eine Steckvorrichtung (23) am Gehäuse (10, 10a, 10b, 10c, 10d, 18, 19, 21, 22) der Steuereinrichtung (1) einsteckbar ist, wobei insbesondere das Gehäuse der Steuereinrichtung eine Ausnehmung (20, 20') zur wenigstens teilweisen, insbesondere vollständigen Aufnahme des Funkmoduls (12, 12') aufweist.

## Claims

1. A medical device (2), comprising an implantable element (3), in particular a heart pump, and a control unit (1) for the implantable element, which is connected to the implantable element by a first connection (4), **characterized in that** the control unit (1) is configured for a predetermined orientation outside and relative a patient's body (6) and comprises a radio module (12, 12'), an antenna (13, 13a, 13b, 13c, 13d, 13e, 13f, 13g) of the radio module (12, 12') being arranged in such a way that the region in which the position of patient's body is provided, as viewed from the control unit (1), is shielded at least partially from the antenna (13, 13a, 13b, 13c, 13d, 13e, 13f, 13g) by electromagnetically shielding, and in particular electrically conducting, parts (10, 10a, 11) of the control unit or the housing (10, 10a, 10b, 10c, 10d, 18, 19, 21, 22) thereof.

2. The medical device according to claim 1, **characterized in that** the antenna is positioned and oriented in such a way that the influence of a patient's body, in the operating state, on the radiation pattern of the antenna is minimized, in particular **in that** the maximum radiation intensity, from the antenna, is situated in a direction that does not affect the position of the patient's body.

3. The medical device according to claim 1 or 2, **characterized in that** the control unit (1) comprises an at least sectionally electrically conducting housing (10, 10a, 10b, 10c, 10d).

4. The medical device according to claim 2 or 3, **characterized in that** the antenna is at least partially arranged within the housing (10, 10a, 10b, 10c, 10d, 18, 19, 21, 22) of the control unit behind a region (10b, 10c, 10d, 18, 19) of the housing which allows radio signals to pass, or behind a window-like recess (17).

5. The medical device according to claim 1 or any one that follows, **characterized in that** the antenna (13, 13a, 13b, 13c, 13d, 13e, 13f, 13g) is at least partially arranged outside the housing (10, 10a, 10b, 10c, 10d, 18, 19) of the control unit.

6. The medical device according to claim 5, **characterized in that** the antenna (13, 13a, 13b, 13c, 13d, 13e, 13f, 13g) extends, at least in sections, substantially parallel to a housing edge of an electrically conducting housing part (10a) of the control unit at a distance of at least 1 mm therefrom.

7. The medical device according to claim 5, **characterized in that** the antenna (13g) has the shape of a dipole, in particular having two closed conductor loops or conductor surfaces.

8. The medical device according to claim 1 or any one that follows, **characterized in that** the housing (10, 10a, 10b, 10c, 10d, 18, 19, 21, 22) of the control unit comprises at least one region (10b, 10c, 10d, 18, 19) that is made of an electrically insulating material that allows electromagnetic waves to pass.

9. The medical device according to claim 8, **characterized in that** an antenna (13, 13a, 13b, 13c, 13d, 13e, 13f, 13g) is arranged within the housing (10, 10a, 10b, 10c, 10d, 18, 19) in or directly behind the region (10b, 10c, 10d, 18, 19, 21, 22) that is made of an electrically insulating material that allows electromagnetic waves to pass.

10. The medical device according to claim 8 or 9, **characterized in that** the, or a, region (21, 22) made of an electrically insulating material that allows electromagnetic waves to pass completely surrounds the electrically conducting region of the housing and/or is tightly joined with the electrically conducting region (10a) of the housing, in particular adhesively bonded thereto, cast thereto or tightly connected by means of an elastomer seal.

11. The medical device according to claim 8, 9 or 10, **characterized in that** the, or a, region (10b, 10c, 10d, 18, 19, 21, 22) of the housing made of an electrically insulating material that allows electromagnetic waves to pass protrudes beyond the contour of the electrically conducting regions (10a) of the housing, an antenna (13, 13a, 13b, 13c, 13d, 13e, 13f, 13g) being arranged in the region protruding beyond the contour of the electrically conducting regions of the housing.

12. The medical device according to claim 1 or any one that follows, **characterized in that** the radio module (12, 12') can be plugged into a plug device (23) on the housing (10, 10a, 10b, 10c, 10d, 18, 19, 21, 22) of the control unit (1), the housing of the control unit in particular including a recess (20, 20') for at least partially, and in particular completely, accommodating the radio module (12, 12').

## Revendications

1. Appareil médical (2) comprenant un élément implantable (3), notamment une pompe cardiaque, et un dispositif de commande (1) pour l'élément implantable, qui est relié au moyen d'une première liaison (4) avec l'élément implantable, **caractérisé en ce que** le dispositif de commande (1) est conçu pour une orientation prédéfinie en dehors et par rapport à un corps de patient (6) et présente un module radio (12, 12'), où une antenne (13, 13a, 13b, 13c, 13d, 13e, 13f, 13g) du module radio (12, 12') est disposée de telle manière que la zone dans laquelle, vu à partir du dispositif de commande (1), la position du corps du patient est prévue, est blindée au moins partiellement par des pièces de blindage électromagnétique, notamment électriquement conductrices (10, 10a, 11) du dispositif de commande ou de son boitier (10, 10a, 10b, 10c, 10d, 18, 19, 21, 22) vis-à-vis de l'antenne (13, 13a, 13b, 13c, 13d, 13e, 13f, 13g).

2. Appareil médical selon la revendication 1, **caractérisé en ce que** l'antenne est positionnée de telle manière que l'effet d'un corps de patient à l'état de fonctionnement est minimisé vis à vis de la caractéristique de rayonnement de l'antenne, notamment **en ce que** le maximum de l'intensité de rayonnement à partir de l'antenne est disposé dans une direction qui ne touche pas la position du corps du patient.

3. Appareil médical selon la revendication 1 ou 2, **caractérisé en ce que** le dispositif de commande (1) présente un boitier conducteur électriquement (10, 10a, 10b, 10c, 10d) au moins par endroits.

4. Appareil médical selon la revendication 2 ou 3, **caractérisé en ce que** l'antenne est disposée au moins partiellement à l'intérieur du boitier (10, 10a, 10b, 10c, 10d, 18, 19, 21, 22) du dispositif de commande derrière une zone (10b, 10c, 10d, 18, 19) du boitier laissant passer les signaux radio ou derrière une cavité (17) de type fenêtre.

5. Appareil médical selon la revendication 1 ou l'une des revendications suivantes, **caractérisé en ce que** l'antenne (13, 13a, 13b, 13c, 13d, 13e, 13f, 13g) est disposée au moins partiellement à l'extérieur du boitier (10, 10a, 10b, 10c, 10d, 18, 19) du dispositif de commande.

6. Appareil médical selon la revendication 5, **caractérisé en ce que** l'antenne (13, 13a, 13b, 13c, 13d, 13e, 13f, 13g) s'étend au moins par endroits de manière essentiellement parallèle par rapport à une arête de boitier d'une partie de boitier (10a) conductrice électriquement du dispositif de commande à une distance d'au moins 1 mm par rapport à celui-ci.

7. Appareil médical selon la revendication 5, **caractérisé en ce que** l'antenne (13g) présente la forme d'un dipôle, notamment avec deux boucles ou surfaces conductrices fermées.

8. Appareil médical selon la revendication 1 ou l'une des revendications suivantes, **caractérisé en ce que** le boitier (10, 10a, 10b, 10c, 10d, 18, 19, 21, 22) du dispositif de commande présente au moins une zone (10b, 10c, 10d, 18, 19) qui est constituée d'un matériau isolant électriquement, perméable aux ondes électromagnétiques.

9. Appareil médical selon la revendication 8, **caractérisé en ce qu'**à l'intérieur du boitier (10, 10a, 10b, 10c, 10d, 18, 19), dans la zone (10b, 10c, 10d, 18, 19, 21, 22) constituée à base du matériau isolant électriquement, perméable aux ondes électromagnétiques, ou immédiatement derrière, il y a une antenne (13, 13a, 13b, 13c, 13d, 13e, 13f, 13g).

10. Appareil médical selon la revendication 8 ou la revendication 9, **caractérisé en ce que** la zone ou une zone (21, 22) constituée à base d'un matériau isolant électriquement, perméable aux ondes électromagnétiques entoure totalement la zone conductrice électriquement du boitier et/ou est reliée de manière étanche, notamment collée, coulée ou reliée au moyen d'un joint élastomère, avec la zone (10a) conductrice électriquement du boitier.

11. Appareil médical selon la revendication 8, 9 ou 10, **caractérisé en ce que** la zone ou une zone (10b, 10c, 10d, 18, 19, 21, 22) constituée à base d'un matériau isolant électriquement, perméable aux ondes électromagnétique du boitier déborde au-delà du contour des zones (10a) conductrices électriquement du boitier, où une antenne (13, 13a, 13b, 13c, 13d, 13e, 13f, 13g) est disposée dans la zone débordant au-delà des zones conductrices électriquement du boitier.

12. Appareil médical selon la revendication 1 ou l'une des revendications suivantes, **caractérisé en ce que** le module radio (12, 12') peut être branché sur un dispositif de connexion (23) sur le boitier (10, 10a, 10b, 10c, 10d, 18, 19, 21, 22) du dispositif de commande (1), où le boitier du dispositif de commande présente en particulier une cavité (20, 20') pour la réception au moins partielle, notamment totale, du module radio (12, 12').
